# EUROPEAN PATENT APPLICATION

(11) **EP 3 960 676 A1**
(43) Date of publication of application: **02.03.2022**
(21) Application number: 20192860.3
(22) Date of filing: 26.08.2020
(51) Int. Cl.: B66B 5/02

(54) **ELEVATOR SMOKE AND FIRE DETECTION SYSTEM**

(71) Applicant: Otis Elevator Company, Farmington, Connecticut 06032 (US)
(72) Inventor: TOUTAOUI, Mustapha, 13507 Berlin (DE)
(74) Representative: Dehns

(57) **Abstract**

An elevator fire detection system (130) for early detection of smouldering and/or burning within an elevator system (100) is provided. A controller (112) is in electronic communication with at least one electronic nose detector (120) which is configured to receive a fluid mixture comprising an odorant, collect sensor data representative of the odorant, compare the sensor data to at least one olfactory reference profile, determine whether the odorant corresponds to the at least one olfactory reference profile and output an indicator signal to the controller (112) to indicate that the detected odorant corresponds to the at least one olfactory reference profile. The controller (112) after receiving the indicator signal effects a safety response action in the elevator system (100).

## Description

### Technical field

The disclosure relates to fire detection systems for use in elevator systems and to methods of early detection of fires in elevator systems.

### Background

It is well known that, in the event of a fire within a building, it is advised not to use a building elevator system in the course of building evacuation. This is because a fire may damage or affect the electronic control of an elevator, trapping passengers or stopping the elevator at a floor where the fire has been detected. Elevator cars and elevator hoistways are also confined spaces that may quickly flood with smoke and suffocate those inside. It is therefore extremely dangerous if a fire originates within an elevator car, for example an electronic fire due to faulty or damaged electronics within a control panel.

Generally, commercially available fire detectors for elevators include smoke particle sensors (ionisation or photoelectric detectors), temperature sensing detectors and flame detectors. However, these devices cannot detect the early stages of a fire since the threshold for detection is often set to be at a high value (e.g. high smoke density or temperatures) to reduce instances of false alarms.

The object of the present invention is thus the early detection of a smoke and fire within elevator systems and effecting an appropriate safety response.

### Summary of Invention

According to a first aspect of the present disclosure, an elevator fire detection system is provided for early detection of smouldering and/or burning within an elevator system. The elevator fire detection system comprises:
at least one electronic nose detector;
a controller in electronic communication with the at least one electronic nose detector and the elevator system;
wherein the at least one electronic nose detector is configured to:
   receive a fluid mixture comprising an odorant;
   collect sensor data representative of the odorant;
   compare the sensor data to at least one olfactory reference profile;
   determine whether the odorant corresponds to the at least one
olfactory reference profile; and
   output an indicator signal to the controller wherein the indicator signal indicates that the detected odorant corresponds to the at least one olfactory reference profile;
wherein upon receipt of the indicator signal the controller is configured to effect a safety response action in the elevator system.

The elevator fire detection system provides early detection of smouldering and/or burning within an elevator system. The smouldering and/or burning may originate from elevator system components, for example electrical and/or electronic components of the elevator system.

In some examples, the at least one olfactory reference profile represents olfactory data for known odorants associated with smouldering and/or burning components of an elevator system. In some examples, the at least one olfactory reference profile may represent olfactory data for known odorants associated with smouldering and/or burning electrical components of the elevator system. For example, the olfactory profile may represent the olfactory data for the known odorants associated with a burning tension element or electrical wiring of the landing or elevator car user control panels.

In some examples at least one electronic nose detector is positioned proximate to elevator system components comprising electrical wiring, electrical components and/or electronic components. For example, in some examples an electronic nose detector may be positioned on an elevator car exterior. In some examples an electronic nose detector may be positioned inside an elevator car, for example, on the ceiling of the elevator car.

In some examples the electronic nose detector may be positioned on the top of the elevator car where the elevator car wiring derived from the different components (e.g. a junction box, door system, safety switches) are collected before sending the information through a traveling cable to the controller located, for example, at the top of the hoistway or on the landing floor.

In some examples an electronic nose detector may be positioned inside an elevator system user control panel positioned inside the elevator car, e.g. elevator car control operating panel (COP) where the elevator car call buttons and displays for run direction and floor level are mounted. In some examples, at least one electronic nose detector may be provided inside the elevator system user control panel inside the elevator car which is positioned to be adjacent to the elevator car doors. In examples where the elevator car is a multi-door elevator car (e.g. a two door elevator car) there may be multiple COP's and one electronic nose detector is positioned in at least one and optionally all, e.g. both. It will be appreciated that a user control panel positioned inside an elevator car may be used by passengers (e.g. users) to register a destination floor for travel. It will be appreciated that additionally or alternatively a user control panel positioned inside the elevator car may perform any number of suitable and/or desirable operations. For example, the user control panel may be used by passengers to send an emergency call or help request.

In some examples an electronic nose detector may be positioned inside an elevator system user control panel positioned on an elevator landing. In some examples, at least one electronic nose detector may be provided inside the elevator system user control panel located in an elevator landing and adjacent to the elevator landing doors. It will be appreciated that a user control panel positioned on a landing may be used by passengers (e.g. users) to register a service request (e.g. automatically or manually) and/or destination request.

In some examples, an electronic nose detector may be provided at a lower portion of the elevator car, for example at an underside of the elevator car where load weighing switches are mounted and wired below the elevator car.

In some examples the at least one electronic nose detector may be positioned in an elevator hoistway of the elevator system. For example, an electronic nose detector may be positioned proximate to the (e.g. electronic) governor and/or proximate to the landing doors on the hoistway side. In some examples a plurality of electronic nose detectors may be positioned at regular or irregular intervals throughout the elevator hoistway of the elevator system.

In some examples, the elevator safety response action may comprise at least one of: activating an alarm, sending a signal to a remote station, stopping an elevator car of the elevator system, and opening elevator car doors, or any suitable and desirable combination thereof.

In some examples, the elevator safety response action may comprise sending a signal to a remote station. In some examples, the elevator safety response action may comprise sending a signal to multiple remote stations. The remote station may be an emergency services department, for example firefighting services (e.g. a fire station) such that a request for assistance is logged. In some examples, the remote station may be a centralised emergency service coordination centre (e.g. 999 or 911 or any other suitable national or local emergency contact number). In some examples, the remote station may be an elevator management server such that the management company is notified that an elevator system requires maintenance or repair. In some examples, the remote station may be a portable maintenance device carried by an engineer located within the building or area such that the elevator unit and/or elevator system may be inspected within a short timeframe e.g. within a few minutes.

In some examples, the elevator safety response action may comprise sending a signal to a remote station where the signal may comprise identifier information. For example, the identifier information may comprise one or more of the following: geographical coordinates indicating the location of the elevator system, location of the electronic nose device which has detected an odorant associated with burning or smouldering, the sensor data representative of the odorant profile and/or the olfactory reference profile that has been determined to correspond to the sensor data, an indication of the degree or extent to which the olfactory reference profile corresponds to the sensor data, and/or an indication or alert as to the severity or stage of the fire. For example a "low severity" alert may indicate that the burning and/or smouldering has been detected at a very low level (e.g. the odorant has a low concentration) and thus the burning and/or smouldering may have just started. In contrast, a "high severity" alert may indicate that the odorant has been detected at high concentrations and thus the burning and/or smouldering is fully established and immediate or prioritised assistance may be required.

In some examples, the elevator safety response action may comprise stopping the elevator car, preferably at the nearest available elevator landing serviced by the elevator system. For example, if an electronic nose detector positioned on an elevator landing detects an odorant which corresponds to an olfactory reference profile the controller may stop the elevator car at the nearest elevator landing which does not correspond to the landing where the odorant was detected. In some examples the elevator controller may configure the elevator car to return to the ground floor or the lowest floor in the building serviced by the elevator system.

In some examples, the elevator safety response action may further comprise opening the elevator car and/or landing doors after the elevator car has been stopped such that passengers are able to disembark and evacuate the building safely.

In some examples, the elevator safety response action may comprise activating an alarm in the elevator car and/or in the building where the elevator system is located (e.g. in the elevator landing(s) or hallway(s)). In some examples, the alarm may be a visual alarm. In some examples, the alarm may be an auditory alarm. For example, an auditory alarm may be a spoken word or phrase such as "please evacuate the elevator".

The electronic nose device of the elevator fire detection system may comprise:
a sensor array configured to receive the fluid mixture comprising the odorant and collect the sensor data representative of the odorant;
a pattern recognition unit configured to receive the sensor data from the sensor array; compare the sensor data to the at least one olfactory reference profile; and determine whether the odorant corresponds to the at least one olfactory reference profile; and
a transmitting unit configured to transmit the indicator signal from the electronic nose detector to the controller.

In some examples, the pattern recognition unit may be in communication with a database in which the at least one olfactory reference profile is stored. In some examples, the database may be stored in the memory of the electronic nose device. In some examples, the database may be stored on a remote server and/or in the cloud such that the pattern recognition unit is in wireless communication with the database.

According to a second aspect of the present disclosure, an elevator system is provided. The elevator system comprises:
a hoistway;
an elevator car disposed within the hoistway and configured for moving between a plurality of landings;
a machine configured to control the movement of the elevator car; and
an elevator fire detection system comprising:
   at least one electronic nose detector;
   a controller in electronic communication with the at least one electronic nose detector and the elevator system;
   wherein the at least one electronic nose detector is configured to:
      receive a fluid mixture comprising an odorant;
      collect sensor data representative of the odorant;
      compare the sensor data to at least one olfactory reference profile;
      determine whether the odorant corresponds to the at least one olfactory reference profile; and
      output an indicator signal to the controller wherein the indicator signal indicates that the detected odorant corresponds to the at least one olfactory reference profile;
   wherein upon receipt of the indicator signal the controller is configured to effect a safety response action in the elevator system.

The elevator system provides early detection of smouldering and/or burning within an elevator system. The smouldering and/or burning may originate from elevator system components, for example electrical and/or electronic components of the elevator system.

In some examples, the at least one olfactory reference profile represents olfactory data for known odorants associated with smouldering and/or burning components of an elevator system. In some examples, the at least one olfactory reference profile may represent olfactory data for known odorants associated with smouldering and/or burning electrical components of the elevator system. For example, the olfactory profile may represent the olfactory data for the known odorants associated with a burning tension element or electrical wiring of the landing or elevator car user control panels.

In some examples at least one electronic nose detector is positioned proximate to elevator system components comprising electrical wiring, electrical components and/or electronic components. For example, in some examples an electronic nose detector may be positioned on an elevator car exterior. In some examples an electronic nose detector may be positioned inside an elevator car, for example, on the ceiling of the elevator car.

In some examples an electronic nose detector may be positioned inside an elevator system user control panel positioned inside the elevator car. In some examples, at least one electronic nose detector may be provided inside the elevator system user control panel inside the elevator car and adjacent to the elevator car doors. It will be appreciated that a user control panel positioned inside an elevator car may be used by passengers (e.g. users) to register a destination floor for travel. It will be appreciated that additionally or alternatively a user control panel positioned inside the elevator car may perform any number of suitable and/or desirable operations. For example, the user control panel may be used by passengers to send an emergency call or help request.

In some examples an electronic nose detector may be positioned inside an elevator system user control panel positioned on an elevator landing. In some examples, at least one electronic nose detector may be provided inside the elevator system user control panel located in an elevator landing and adjacent to the elevator landing doors. It will be appreciated that a user control panel positioned on a landing may be used by passengers (e.g. users) to register a service request (e.g. automatically or manually) and/or destination request.

In some examples the at least one electronic nose detector may be positioned in an elevator hoistway of the elevator system. For example, an electronic nose detector may be positioned proximate to the (e.g. electronic) governor and/or proximate to the landing doors on the hoistway side. In some examples a plurality of electronic nose detectors may be positioned at regular or irregular intervals throughout the elevator hoistway of the elevator system.

In some examples, the elevator safety response action may comprise at least one of activating an alarm, sending a signal to a remote station, stopping an elevator car of the elevator system, and opening elevator car doors or any suitable and desirable combination thereof.

In some examples, the elevator safety response action may comprise sending a signal to a remote station. In some examples, the elevator safety response action may comprise sending a signal to multiple remote stations. The remote station may be an emergency services department, for example firefighting services (e.g. a fire station) such that a request for assistance is logged. In some examples, the remote station may be a centralised emergency service coordination centre (e.g. 999 or 911 or any other suitable national or local emergency contact number). In some examples, the remote station may be an elevator management server such that the management company is notified that an elevator system requires maintenance or repair.

In some examples, the elevator safety response action may comprise sending a signal to a remote station where the signal may comprise identifier information. For example, the identifier information may comprise geographical coordinates indicating the location of the elevator system, the electronic nose device location which has detected an odorant associated with burning or smouldering, the sensor data representative of the odorant profile and/or the olfactory reference profile that has been determined to correspond to the sensor data, an indicator of the degree or extent to which the olfactory reference profile corresponds to the sensor data, and/or an indication or alert as to the severity or stage of the fire. For example a "low severity" alert may indicate that the burning and/or smouldering has been detected at a very low level (e.g. the odorant has a low concentration) and thus the burning and/or smouldering may have just started. In contrast, a "high severity" alert may indicate that the odorant has been detected at high concentrations and thus the burning and/or smouldering is fully established and immediate or prioritised assistance may be required.

In some examples, the elevator safety response action may comprise stopping the elevator car, optionally at the nearest available elevator landing serviced by the elevator system. For example, if an electronic nose detector positioned on an elevator landing detects an odorant which corresponds to an olfactory reference profile the controller may stop the elevator car at the nearest elevator landing which does not correspond to the landing where the odorant was detected. In some examples the elevator controller may configure the elevator car to return to the ground floor or the lowest floor in the building serviced by the elevator system.

In some examples, the elevator safety response action my further comprise opening the elevator car and/or landing doors after the elevator car has been stopped such that passengers are able to disembark and evacuate the building safely.

In some examples, the elevator safety response action may comprise activating an alarm in the elevator car and/or in the building where the elevator system is located (e.g. in the elevator landing hallways). In some examples, the alarm may be a visual alarm. In some examples, the alarm may be an auditory alarm. For example, an auditory alarm may be a spoken word or phrase such as "please evacuate the elevator".

According to a third aspect of the present invention, a method is provided for the early detection of burning and/or smouldering. The method comprises:
receiving a fluid mixture comprising an odorant at an electronic nose device;
collecting sensor data representative of the odorant using the electronic nose device;
comparing the sensor data to at least one olfactory reference profile;
determining whether the odorant corresponds to the at least one olfactory reference profile;
outputting from the electronic nose device an indicator signal to a controller; and
effecting a safety response action using the controller which is configured to be in communication with the electronic nose device and the elevator system.

The method provides early detection of smouldering and/or burning within an elevator system. The smouldering and/or burning may originate from elevator system components, for example electrical and/or electronic components of the elevator system.

In some examples, the at least one olfactory reference profile represents olfactory data for known odorants associated with smouldering and/or burning components of an elevator system. In some examples, the at least one olfactory reference profile may represent olfactory data for known odorants associated with smouldering and/or burning electrical components of the elevator system. For example, the olfactory profile may represent the olfactory data for the known odorants associated with a burning tension element or electrical wiring of the landing or elevator car user control panels.

In some examples at least one electronic nose detector is positioned proximate to elevator system components comprising electrical wiring, electrical components and/or electronic components. For example, in some examples an electronic nose detector may be positioned on an elevator car exterior. In some examples an electronic nose detector may be positioned inside an elevator car, for example, on the ceiling of the elevator car.

In some examples an electronic nose detector may be positioned inside an elevator system user control panel positioned inside the elevator car. In some examples, at least one electronic nose detector may be provided inside the elevator system user control panel inside the elevator car and adjacent to the elevator car doors. It will be appreciated that a user control panel positioned inside an elevator car may be used by passengers (e.g. users) to register a destination floor for travel. It will be appreciated that additionally or alternatively a user control panel positioned inside the elevator car may perform any number of suitable and/or desirable operations. For example, the user control panel may be used by passengers to send an emergency call or help request.

In some examples an electronic nose detector may be positioned inside an elevator system user control panel positioned on an elevator landing. In some examples, at least one electronic nose detector may be provided inside the elevator system user control panel located in an elevator landing and adjacent to the elevator landing doors. It will be appreciated that a user control panel positioned on a landing may be used by passengers (e.g. users) to register a service request (e.g. automatically or manually) and/or destination request.

In some examples the at least one electronic nose detector may be positioned in an elevator hoistway of the elevator system. For example, an electronic nose detector may be positioned proximate to the (e.g. electronic) governor and/or proximate to the landing doors on the hoistway side. In some examples a plurality of electronic nose detectors may be positioned at regular or irregular intervals throughout the elevator hoistway of the elevator system.

In some examples, the elevator safety response action may comprise at least one of activating an alarm, sending a signal to a remote station, stopping an elevator car of the elevator system, and opening elevator car doors or any suitable and desirable combination thereof.

In some examples, the elevator safety response action may comprise sending a signal to a remote station. In some examples, the elevator safety response action may comprise sending a signal to multiple remote stations. The remote station may be an emergency services department, for example firefighting services (e.g. a fire station) such that a request for assistance is logged. In some examples, the remote station may be a centralised emergency service coordination centre (e.g. 999 or 911 or any other suitable national or local emergency contact number). In some examples, the remote station may be an elevator management server such that the management company is notified that an elevator system requires maintenance or repair.

In some examples, the elevator safety response action may comprise sending a signal to a remote station where the signal may comprise identifier information. For example, the identifier information may comprise geographical coordinates indicating the location of the elevator system, the electronic nose device location which has detected an odorant associated with burning or smouldering, the sensor data representative of the odorant profile and/or the olfactory reference profile that has been determined to correspond to the sensor data, an indicator of the degree or extent to which the olfactory reference profile corresponds to the sensor data, and/or an indication or alert as to the severity or stage of the fire. For example a "low severity" alert may indicate that the burning and/or smouldering has been detected at a very low level (e.g. the odorant has a low concentration) and thus the burning and/or smouldering may have just started. In contrast, a "high severity" alert may indicate that the odorant has been detected at high concentrations and thus the burning and/or smouldering is fully established and immediate or prioritised assistance may be required.

In some examples, the elevator safety response action may comprise stopping the elevator car, preferably at the nearest available elevator landing serviced by the elevator system. For example, if an electronic nose detector positioned on an elevator landing detects an odorant which corresponds to an olfactory reference profile the controller may stop the elevator car at the nearest elevator landing which does not correspond to the landing where the odorant was detected. In some examples the elevator controller may configure the elevator car to return to the ground floor or the lowest floor in the building serviced by the elevator system.

In some examples, the elevator safety response action may further comprise opening the elevator car and/or landing doors after the elevator car has been stopped such that passengers are able to disembark and evacuate the building safely.

In some examples, the elevator safety response action may comprise activating an alarm in the elevator car and/or in the building where the elevator system is located (e.g. in the elevator landing hallways). In some examples, the alarm may be a visual alarm. In some examples, the alarm may be an auditory alarm. For example, an auditory alarm may be a spoken word or phrase such as "please evacuate the elevator".

### Detailed description

The present invention is illustrated by way of example and not limited in the accompanying figures in which like reference numerals indicate similar elements.
Figure 1 is a schematic illustration of an elevator system that may employ various examples of the present disclosure;
Figure 2 shows a side view schematic illustration of an elevator system that may employ various examples of the present disclosure;
Figure 3 shows a schematic illustration of an electronic nose detector according to an example of the present disclosure;
Figure 4 shows a method for operating a fire detection system according to an example of the present disclosure; and
Figure 5 shows a method of operating a fire detection system according to an example of the present disclosure.

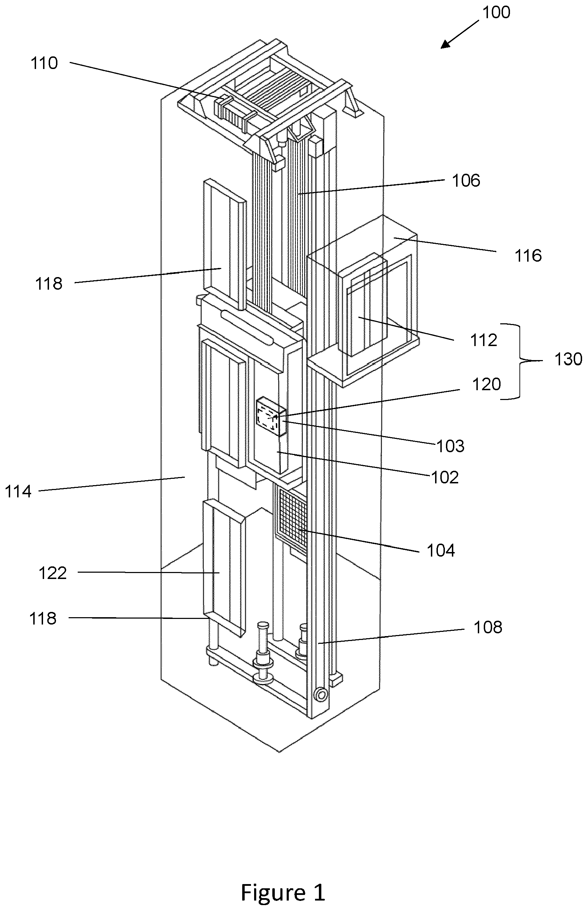

Figure 1 is a perspective view of an elevator system 100 including an elevator car 102, a counterweight 104, a tension member 106, a guide rail 108, a machine 110, and a controller 112. The elevator car 102 and counterweight 104 are connected to each other by the tension member 106. The tension member 106 may include or be configured as, for example, ropes, steel, cables, and/or coated steel belts. The counterweight 104 is configured to balance a load of the elevator car 102 and facilitates movement of the elevator car 102. The elevator car 102 and the counterweight 104 move concurrently and in an opposite direction within an elevator hoistway 114 and along the guide rail 108.

The tension member 106 engages the machine 110 which is part of the overhead structure of the elevator system 100. The machine 110 is configured to control movement between the elevator car 102 and the counterweight 104.

The controller 112 is located, as shown, in a controller room 116 of the elevator hoistway 114 and is configured to control the operation of the elevator system 100 and particularly the elevator car 102. For example, the controller 112 may provide drive signals to the machine 110 to control the acceleration, deceleration, levelling, stopping, etc. of the elevator car 102. The controller 112 is configured to receive signals from an electronic nose detector 120. The controller 112 and the electronic nose detector 120 are configured for wireless data transmission. Alternatively there is a wired data connection between the controller 112 and the electronic nose detector 120.

When moving up or down within the elevator hoistway 114 along the guide rail 108, the elevator car 102 may stop at one or more landings 118 as controlled by the controller 112. Although shown in a controller room 116, it will be appreciated that the controller 112 can be located and/or configured in other locations or positions within the elevator system 100. In one embodiment, the controller 112 may be located remotely or in the cloud.

The machine 110 may include a motor or similar driving mechanism. In accordance with embodiments of the disclosure, the machine 110 is configured to include an electrically driven motor. The power supply for the motor may be any power source, including a power grid, which, in combination with other components, is supplied to the motor. The machine 110 may include a traction sheave that imparts force to tension member 106 to move the elevator car 102 within the elevator hoistway 114.

The machine 110 may include a motor or similar driving mechanism. In accordance with embodiments of the disclosure, the machine 110 is configured to include an electrically driven motor. The power supply for the motor may be any power source, including a power grid, which, in combination with other components, is supplied to the motor. The machine 110 may include a traction sheave that imparts force to tension member 106 to move the elevator car 102 within the elevator hoistway 114.

Although shown and described with a tension member 106, elevator systems that employ other methods and mechanisms of moving an elevator car 102 within an elevator hoistway 114 may employ embodiments of the present disclosure. For example, embodiments may be employed in ropeless elevator systems using a linear motor to impart motion to an elevator car. Embodiments may also be employed in ropeless elevator systems using a hydraulic lift to impart motion to an elevator car. Figure 1 is merely a non-limiting example presented for illustrative and explanatory purposes.

The controller 112 and the electronic nose detector 120 form a fire detection system 130.

In the example of Figure 1, the electronic nose detector 120 is provided within the elevator car 102, within or adjacent to an elevator car control panel 103 (e.g. the panel located inside an elevator car for a passenger to register a destination floor). The electronic nose detector 120 is configured to detect odorant released from electrical cables and/or elements of wiring looms which are released when such a component begins to overheat and an outer plastic coating begins to smoulder and melt. In this way, the electronic nose detector 120 can determine if any of the electrical cables and/or elements of wiring looms provided in the elevator control panel 103 begin to smoulder or melt due to malfunction before too much damage can occur.

Upon determination that an odorant detected by the electronic nose detector 120 is associated with the burning or smouldering of electrical cables and/or components, the electronic nose detector 120 sends a signal to the controller 112 to effect a predetermined and/or desired safety response action. For example, the controller 112 may be configured for communication with an alarm or alarm system and/or other warning systems within the elevator system and/or building such that upon receipt of a signal from the electronic nose detector 120 indicating that a smouldering, melting or burning cable has been detected an alarm may be raised and the building may need to be evacuated. Alternatively or in addition, the controller 112 may be configured for communication with a remote station, for example to call emergency services or send a message to the elevator management company that the elevator system is in need of maintenance. In some examples, the safety response action may involve the controller 112 controlling the elevator system 100 such that the elevator car 102 stops at the next viable landing 118 to allow passengers to evacuate (e.g. the elevator doors and/or landing doors 122 are opened when the landing is reached).

Whilst Figure 1 shows only one electronic nose detector 120, in an alternative example, the fire detection system 130 may comprise multiple electronic nose detectors 120, provided at suitable locations within the elevator system 100, for example within and/or on an exterior of the elevator car 102.

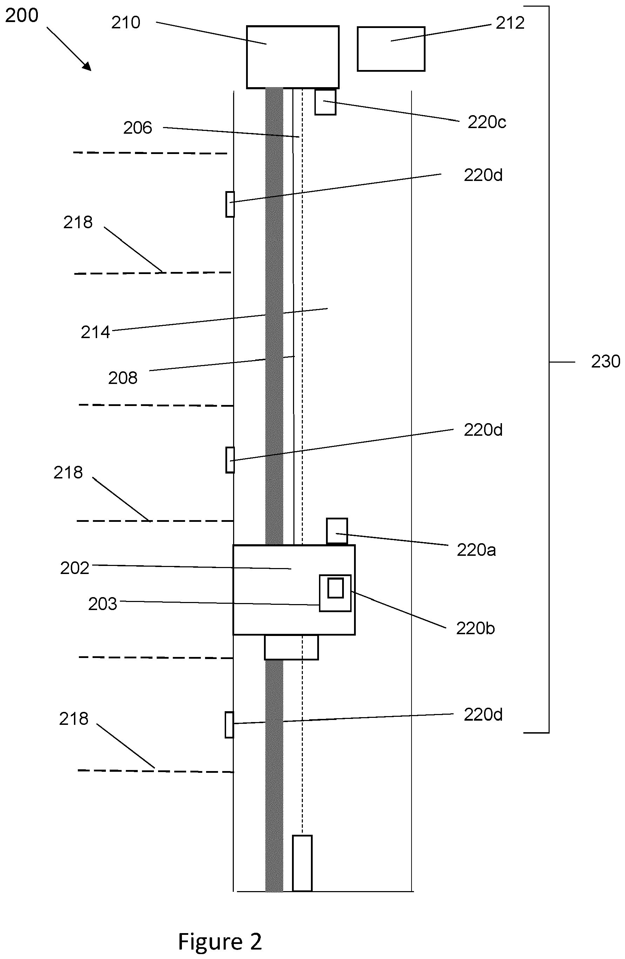

Figure 2 shows a schematic representation of an elevator system 200 in accordance with another example of the present invention when viewed from the side. Similarly to the example shown in Figure 1, the elevator system 200 includes an elevator car 202, a tension member 206, a guide rail 208, a machine 210, and a controller 212. The controller 212 is configured to receive signals from a plurality of electronic nose detectors 220a-220d located throughout the elevator system 200. As shown in Figure 2, an electronic nose detector 220a is positioned on the exterior of the elevator car 202 which may be used to detect smouldering or melting of electrical components exterior to the elevator car 202. Another electronic nose detector 220b is shown positioned inside the elevator car 202, optionally within or adjacent to an elevator car control panel 203 (e.g. the panel located inside an elevator car 202 for a passenger to register a destination floor request). A hoistway electronic nose detector 220c is shown positioned inside the elevator hoistway 214 to detect burning, melting or smouldering of components other than those associated with the elevator car 102. A landing electronic nose detector 220d is shown positioned exterior to the elevator hoistway 214 in each landing 218 serviced by the elevator system 200. Optionally, each landing electronic nose detector 220d is positioned within a landing call control panel (not shown) or system associated with the elevator system 200 (e.g. the control system used by a user to initiate a call request). The hall call control panel may be automatic (e.g. a hall call is sent to the elevator system automatically after detecting a passenger within the landing area) or manual (e.g., the passenger presses a service request button).

In the example of Figure 2, it will be appreciated that a fire detection system 230 includes the controller 212 and the plurality of electronic nose detectors 220, 220a, 220b, 220c, 220d.

It will thus be appreciated that the at least one electronic nose detector 120, 220a, 220b, 220c, 220d may be positioned in any suitable and desirable place within an elevator system 100, 200. Optionally, the or each electronic nose detector 120, 220a, 220b, 220c, 220d is positioned proximate to any component of the elevator system 100, 200 that comprises cabling (e.g. the tension member) or electronic wiring and/or electronic components (e.g. control panels). Thus, optional examples of the invention comprise a plurality of electronic nose detectors 120, 220a, 220b, 220c, 220d positioned throughout the elevator system 100, 200.

In the early stages of a cable fire, for example an electronic fire due to faulty or damaged wiring, a characteristic "smell" (e.g. an olfactory profile) of burning or smouldering associated with the material being burnt may be observed before physically observable signs of flames or smoke are detectable. Olfaction can thus provide a means of far earlier detection with greater sensitivity that other forms of fire detectors. Electronic nose detectors 120, 220a, 220b, 220c, 220d mimic olfaction using an array of chemical sensors that are able to detect various "smells" and determine whether the source of the smell corresponds to the burning olfaction profile of various elevator system components.

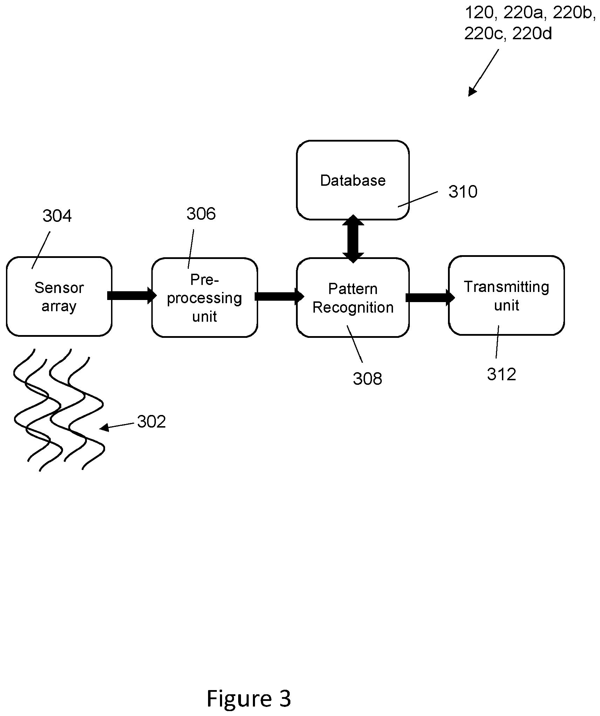

Figure 3 shows schematically a schematic illustration of an electronic nose detector 120, 220a, 220b, 220c, 220d according to an example of the present disclosure. The electronic nose detector 120, 220a, 220b, 220c, 220d comprises a sensor array 304 (for the detection of odorants 302), a pre-processing unit 306, a pattern recognition system 308, an olfactory reference database 310 and a transmitting unit 312. In some examples, after an odorant 302 is detected by the sensor array 304, the sensor array 304 sends the sensor data (e.g. the data representative of the odorant detected) to the pre-processing unit 306 where the data is pre-processed, e.g. to minimise signal data noise. After pre-processing the pre-processing unit 306 transmits the pre-processed signal data to the pattern recognition unit 308 where the signal data is compared to reference olfactory profiles (e.g. olfactory profiles known to be associated with electric cable burning or smouldering) stored within the olfactory reference database 310. If the pattern recognition unit 308 determines that the signal data (representative of the odorant) corresponds to a reference olfactory profile of a burning or smouldering cable, the pattern recognition unit 306 sends a signal to the transmitting unit 312 which transmits a signal to the controller 112, 212 to effect a desired safety response.

In the example of Figure 3, the olfactory reference database 310 is stored locally in a memory provided in the electronic nose detector 120, 220a, 220b, 220c, 220d. In some embodiments (not shown) the olfactory reference database 310 is stored remotely or in the cloud and is in (e.g. wired or wireless) communication with the electronic nose detector 120, 220a, 220b, 220c, 220d.

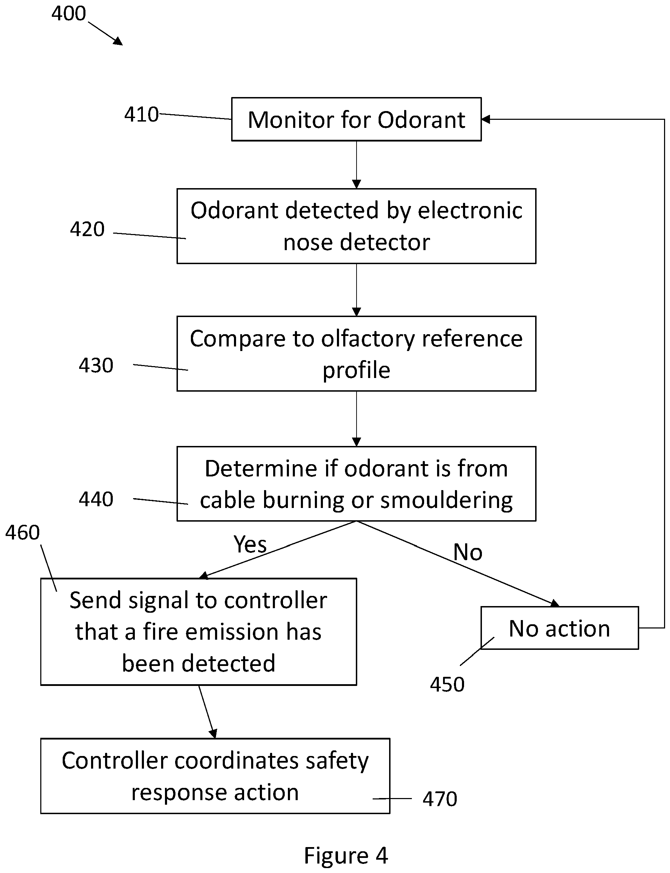

Figure 4 shows a method 400 for the early detection of a fire using the fire detection system 130, 230 of the present disclosure. The method 400 comprises, in step 410, continuously monitoring for an odorant with the sensor array 304 of the electronic nose detector 120, 220a, 220b, 220c, 220d. In step 420, when an odorant 302 is detected by the sensor array 304, the pattern recognition system 308 of the electronic nose detector 120, 220a, 220b, 220c, 220d compares the sensor data representative of the detected odorant to an (at least one) olfactory reference profile. In step 430, the pattern recognition system 308 thus determines whether the odorant corresponds to the reference profile such that the odorant may be determined to be from a burning or smouldering cable or other component. If it is determined that the odorant 302 does not correspond to a reference olfactory profile, no further action is taken (step 450) and the method returns to monitoring for an odorant (step 410). Alternatively, if it is determined that the odorant 302 does correspond to a reference olfactory profile, in step 460, the transmitting unit 312 of the electronic nose detector 120, 220a, 220b, 220c, 220d, sends a signal to the controller 112, 212 to indicate that (e.g. the early stages of) a fire (e.g. burning or smouldering) has been detected. Upon receiving a signal indicating the early stages of a fire, the controller 112, 212 effects at least one safety response action in step 470.

It will be appreciated that the safety response action may be any suitable and/or desirable action. For example, the safety response action may include activating an alarm; automatically sending a message to a remote station such as fire emergency services or a conveyance management company; controlling the elevator system 100, 200 such that the elevator car 102 is stopped at the next landing 118 (or floor); and opening the elevator doors to allow passengers to safely evacuate.

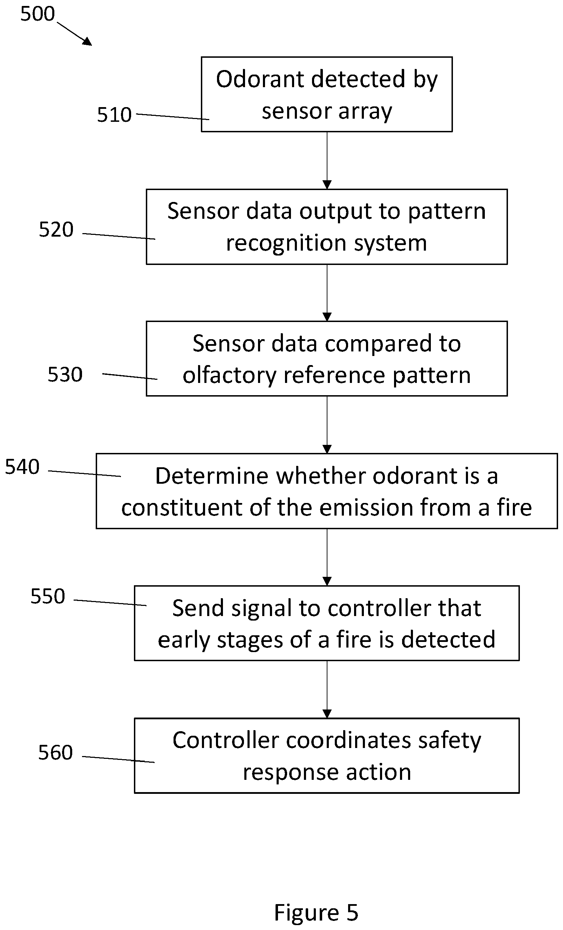

Figure 5 shows a method 500 for the early detection of a fire using the fire detection system 130, 230 in accordance with another example of the present disclosure. In step 510, the sensor array 304 of the electronic nose detector 120, 220a, 220b, 220c, 220d detects an odorant 302. In step 520, the sensor array 304 outputs sensor data representative of the olfaction profile of the odorant 302 to the pattern recognition system 308 of the electronic nose detector 120, 220 a, 220b, 220c, 220d. In step 530, the pattern recognition system 308 compares the olfactory profile of the odorant 302 to a plurality of olfactory reference profiles stored within the olfactory reference database 310. In some embodiments the olfactory reference database 310 is stored locally in a memory provided in the electronic nose detector 120, 220a, 220b, 220c, 220d, e.g. the database comprises a set number of olfactory reference patterns stored in the memory prior to installation within the elevator system. In some embodiments the olfactory reference database 310 is stored remotely or in the cloud and is in (e.g. wired or wireless) communication with the electronic nose detector 120, 220a, 220b, 220c, 220d. In some embodiments the olfactory reference database may be (e.g. manually or automatically) updated with new or updated olfactory patterns at (e.g. at regular or irregular) intervals via wireless or wired communication.

In step 540, it is determined that the odorant 302 corresponds to a reference olfactory profile the transmitting unit 315 of the electronic nose detector 120, 220 a, 220b, 220c, 220d, sends a signal to the controller 112, 212 to indicate that (e.g. the early stages of) a fire (e.g. burning or smouldering) has been detected (step 550). In step 560, upon receiving a signal indicating the early stages of a fire, the controller 112, 212 effects at least one safety response action, as outlined above with reference to Figure 4.

It will be appreciated by those skilled in the art that the disclosure has been illustrated by describing one or more specific aspects thereof, but is not limited to these aspects; many variations and modifications are possible within the scope of the accompanying claims. The various embodiments shown have features which are interchangeable with each other depending on the system.

## Claims

1. An elevator fire detection system (130, 230) for early detection of smouldering and/or burning within an elevator system (100, 200), the elevator fire detection system comprising:
at least one electronic nose detector (120, 220a, 220b, 220c, 220d);
a controller (112, 212) in electronic communication with the at least one electronic nose detector (120, 220a, 220b, 220c, 220d) and the elevator system (100, 200);
wherein the at least one electronic nose detector (120, 220a, 220b, 220c, 220d) is configured to:
receive a fluid mixture comprising an odorant (302);
collect sensor data representative of the odorant (302);
compare the sensor data to at least one olfactory reference profile;
determine whether the odorant (302) corresponds to the at least one olfactory reference profile; and
output an indicator signal to the controller (112, 212) wherein the indicator signal indicates that the detected odorant corresponds to the at least one olfactory reference profile;
wherein upon receipt of the indicator signal the controller (112, 212) is configured to effect a safety response action in the elevator system.

2. The elevator fire detection system (130, 230) of claim 1, wherein the at least one olfactory reference profile represents olfactory data for known odorants associated with smouldering and/or burning components of the elevator system (100, 200).

3. The elevator fire detection system (130, 230) of claim 1 or claim 2, wherein the at least one electronic nose detector (120, 220a, 220b, 220c, 220d) is positioned proximate to elevator system components comprising electrical wiring, electrical components and/or electronic components.

4. The elevator fire detection system (130, 230) of claim 1, 2 or 3, wherein at least one electronic nose detector (120, 220a, 220b, 220c, 220d) is positioned on an elevator car (102, 202) exterior and/or inside an elevator car (102, 202).

5. The elevator fire detection system (130, 230) of any preceding claim, wherein at least one of the electronic nose detectors (120, 220a, 220b, 220c, 220d) is positioned inside an elevator system user control panel;
wherein the elevator system user control panel is positioned inside an elevator car (102, 202) and/or in an elevator landing hall (118, 218) proximate to an elevator landing door (122).

6. The elevator fire detection system (130, 230) of any preceding claim, wherein the safety response action comprises at least one of activating an alarm, sending a signal to a remote station, stopping an elevator car (102, 202) of the elevator system (100, 200), and opening the elevator car doors.

7. The elevator fire detection system (130, 230) of any previous claim, wherein the safety response comprises activating an alarm; wherein the alarm may be a visual alarm and/or an auditory alarm.

8. The elevator fire detection system (130, 230) of any previous claim, wherein the electronic nose device (120, 220a, 220b, 220c, 220d) comprises:
a sensor array (304) configured to receive the fluid mixture comprising the odorant (302) and collect the sensor data representative of the odorant (302);
a pattern recognition unit (306) configured to receive the sensor data from the sensor array (304); compare the sensor data to the at least one olfactory reference profile; and determine whether the odorant corresponds to the at least one olfactory reference profile; and
a transmitting unit (310) configured to transmit the indicator signal from the electronic nose detector (120, 220a, 220b, 220c, 220d) to the controller (112, 212).

9. An elevator system (100, 200) comprising:
a hoistway (114, 214);
an elevator car (102, 202) disposed within the hoistway (114, 214) and configured for moving between a plurality of landings (118, 218);
a machine (110, 210) configured to control the movement of the elevator car (102, 202); and
the elevator fire detection system (130, 230) of any previous claim.

10. A method (400) for the early detection of burning and/or smouldering in an elevator system (100, 200), the method comprising:
receiving a fluid mixture (410) comprising an odorant (302) at an electronic nose device (120, 220a, 220b, 220c, 220d) ;
collecting sensor data (420) representative of the odorant (302) using the electronic nose device (120, 220a, 220b, 220c, 220d);
comparing the sensor data (430) to at least one olfactory reference profile;
determining (440) whether the odorant (302) corresponds to the at least one olfactory reference profile;
outputting (460) from the electronic nose device (120, 220a, 220b, 220c, 220d) an indicator signal to a controller (112, 212); and
effecting a safety response action (470) using the controller (112, 212) which is configured to be in communication with the electronic nose device (120, 220a, 220b, 220c, 220d) and the elevator system (100, 200).

11. The method (400) of claim 10, wherein the at least one olfactory reference profile represents olfactory data for known odorants associated with smouldering and/or burning components of the elevator system (100, 200).

12. The method (400) of claim 10 or claim 11, wherein the at least one electronic nose detector (120, 220a, 220b, 220c, 220d) is positioned proximate to elevator system components comprising electrical wiring, electrical components and/or electronic components.

13. The method (400) of claim 10, 11 or 12 wherein at least one electronic nose detector (120, 220a, 220b, 220c, 220d) is positioned on an elevator car (102, 202) exterior and/or inside an elevator car (102, 202).

14. The method (400) of any previous claim, wherein the safety response action comprises at least one of activating an alarm, sending a signal to a remote station, stopping the elevator car (102, 202) and opening the elevator car doors.

15. The method of any previous claim, wherein the safety response comprises activating an alarm; wherein the alarm may be a visual alarm and/or an auditory alarm.
